# EUROPEAN PATENT APPLICATION

(11) **EP 4 104 779 A1**
(43) Date of publication of application: **21.12.2022**
(21) Application number: 21179800.4
(22) Date of filing: 16.06.2021
(51) Int. Cl.: A61B 17/34, A61B 34/00, A61B 34/30, A61B 34/37, A61B 90/00, A61B 17/00

(54) **SURGICAL ROBOTIC SYSTEM FOR EYE SURGERY AND CONTROL OF THE SAME**

(71) Applicant: Preceyes B.V., 5612 AP Eindhoven (NL)
(72) Inventor: BEELEN, Maarten Joannes, 5612 AP Eindhoven (NL); SMIT, Jorrit, 5612 AP Eindhoven (NL); NAUS, Gerrit Jacobus Lambertus, 5612 AP Eindhoven (NL); MEENINK, Hildebert Christiaan Matthijs, 5612 AP Eindhoven (NL); DE SMET, Marc Joseph Dominique, 5612 AP Eindhoven (NL)
(74) Representative: DeltaPatents B.V.

(57) **Abstract**

A surgical robotic system is provided for use in an ocular surgical procedure. The system comprises a surgical arm (080) comprising a movable arm part (082), which comprises an instrument connector for mounting of a surgical instrument (119) having a longitudinal axis. The movable arm part (082) has at least one degree of freedom to enable longitudinal movement of the surgical instrument (119) along its longitudinal axis, towards or away from an ocular surgical target (123). The system comprises a sensor (030), user interface (020), actuator (060) and processor subsystem (040). The processor subsystem (040) is configured to obtain sensor data (032) from the sensor (030) at an initial position of the surgical instrument (119), determine an initial distance between the surgical instrument (119) and the surgical target (123), obtain data indicating a target position of the surgical instrument (119), determine a displacement distance, output a sensory-perceptible representation of the displacement distance, receive a confirmation signal from the user, and upon receiving the confirmation signal, control the actuator (060) to actuate the movable arm part (082) to effect a single movement of the surgical instrument (119) over the displacement distance.

## Description

### FIELD OF THE INVENTION

The invention relates to a surgical robotic system for use in an ocular surgical procedure. The invention further relates to a method for controlling a surgical robotic system during use in an ocular surgical procedure, and to a computer program product comprising instructions for causing a processor system to perform the method.

### BACKGROUND ART

Ocular surgical procedures increasingly involve the use of surgical robotic systems. Rather than operating entirely autonomously, such surgical robotic systems are typically at least in part under the control of a human operator, for example, to control the movement of a surgical instrument mounted to a surgical arm of the surgical robotic system. As such, surgical robotic systems may assist the human operator in performing a surgical procedure.

Robotic assistance can improve the manipulation precision of moving a surgical instrument. Ocular surgery requires a particularly high degree of precision, given the complexity and size of the tissues and structures found in or around an eye. Robotic systems, however, provide such precision in absolute coordinates, rather than relative to the tissue. Fluctuations or movement in the tissue, for example, reduce the precision offered by the robotic assistance. Moreover, a surgeon operating the robot has only limited visibility of the surgical field through the microscope. In particular, depth perception becomes challenging.

Existing surgical robotic systems exist, which may be provided with a surgical arm which comprises a movable arm part, with the movable arm part comprising an instrument connector for mounting of a surgical instrument. Accordingly, the surgical instrument may be positioned by the surgical arm. A human-machine interface may be provided for receiving positioning commands from a human operator for controlling the movement of the surgical instrument. An actuator may be provided for actuating the movable arm part to effect the movement of the surgical instrument in accordance with the positioning commands provided by the human operator. Examples of this approach may be found in the field of tele-operation, where a human operator may operate a master device, e.g., a motion controller, to provide positioning commands for a slave device, e.g., the aforementioned surgical arm.

However, such systems do not reliably provide the level of precision necessary for ocular surgical procedures, and typically alleviate the problem by processing the position commands received from the user, such as to reduce the effects of a hand tremor.

In US20190110682 A1, the use of an optical coherence tomography (OCT) sensor has been introduced in a vitreoretinal surgery application in order to detect when a surgical injector penetrates a desired tissue layer of the eye. An OCT sensor is used to scan a surgical target. An indication is received from a user, such as to identify a tissue layer for receiving an injection or to confirm an automatic injection.

### SUMMARY OF THE INVENTION

A problem of completely automated movement of a surgical instrument towards a surgical target is that such movement may be based on noisy or otherwise unreliable sensor data. Users, typically surgeons, have historically been apprehensive of adopting fully automated procedures for this reason. For example, if a surgical target is located in a subretinal location, the movement of the eye or of the surgical instrument may make sensor measurements indicating the position of the surgical target less reliable, or if a sensor is partially or fully obscured, e.g. by debris, introducing a risk of the surgical instrument puncturing an unintended tissue or organ.

One of the objects of the invention is to obtain a surgical robotic system and/or method for controlling a surgical robotic system which enables the user to control the displacement of the surgical instrument, whilst still maintaining the advantages of automated movement, including reduced hand shaking or tremor effects. Providing the user with the control over the displacement of the surgical instrument also addresses the inherent apprehension of users to adopt automated procedures. This combination of automated movement with user control over instrument displacement improves the reliability of the surgical robotic system.

A first aspect of the invention provides a surgical robotic system for use in an ocular surgical procedure, comprising:
- a surgical arm comprising a movable arm part, the movable arm part comprising an instrument connector for mounting of a surgical instrument having a longitudinal axis, the movable arm part having at least one degree of freedom to enable longitudinal movement of the surgical instrument along the longitudinal axis of the surgical instrument towards or away from an ocular surgical target;
- a sensor configured to obtain sensor data indicating a distance between the surgical instrument and the surgical target;
- a user interface configured to receive user input from a user and to output sensory perceptible output to the user;
- an actuator configured and arranged for actuating the movable arm part to effect the longitudinal movement of the surgical instrument;
- a processor subsystem configured to:
   - at an initial position of the surgical instrument, obtain sensor data from the sensor and determine an initial distance between the surgical instrument and the surgical target based on the sensor data;
   - obtain data indicative of a target position of the surgical instrument relative to the surgical target;
- determine a displacement distance for the surgical instrument based on the sensor data and the target position;
- output, via the user interface, a sensory-perceptible representation of the determined displacement distance;
- receive, via the user interface, a confirmation signal from the user; and
- upon receiving the confirmation signal, control the actuator to actuate the movable arm part to effect a single movement of the surgical instrument along the longitudinal axis over the determined displacement distance.

In a further aspect of the invention, a computer-implemented method is provided for controlling a surgical robotic system during use in an ocular surgical procedure, the surgical robotic system comprising a surgical arm, the surgical arm comprising a movable arm part, the movable arm part comprising an instrument connector for mounting of a surgical instrument having a longitudinal axis, the movable arm part having at least one degree of freedom to enable longitudinal movement of the surgical instrument along the longitudinal axis of the surgical instrument towards or away from an ocular surgical target, the surgical robotic system further comprising an actuator configured and arranged for actuating the movable arm part to effect the longitudinal movement of the surgical instrument, the method comprising:
- at an initial position of the surgical instrument, obtaining sensor data indicating a distance between the surgical instrument and the surgical target;
- determining an initial distance between the surgical instrument at the initial position and the surgical target based on the sensor data;
- obtaining data indicative of a target position of the surgical instrument relative to the surgical target;
- determine a displacement distance for the surgical instrument based on the sensor data and the target position;
- outputting, to the user, a sensory-perceptible representation of the displacement distance;
- receiving, from the user, a confirmation signal; and
- upon receiving the confirmation signal, controlling the actuator to actuate the movable arm part to effect a single movement of the surgical instrument along the longitudinal axis over the displacement distance.

In a further aspect of the invention, a computer program product is provided comprising instructions for causing a processor system to perform the method.

The above aspects of the invention involve a surgical robotic system which comprises a surgical arm. The surgical arm comprises a movable arm part, with the movable arm part comprising an instrument connector for mounting of a surgical instrument. The surgical instrument has a longitudinal axis, typically passing through a tip of the surgical instrument. The movable arm part has at least one Degree-of-Freedom (DoF) to enable longitudinal movement of the surgical instrument along the longitudinal axis of the surgical instrument towards a surgical target. The surgical target may for example be a tissue layer or a surface of such a layer, such as the surface of an upper retinal layer. It is noted that the movable arm part may have exactly one DoF aligned with the longitudinal axis of the surgical instrument to enable said longitudinal movement. However, the movable arm part may also have multiple DoFs enabling said longitudinal movement yet without any of the DoFs having to be individually aligned with the longitudinal axis. It is noted that surgical arms having the functionality described in this paragraph are known per se from the field of medical robotics, and also known as instrument manipulators, robotic arms, surgical robot slave devices, etc.

A user interface is provided for receiving user input, such as positioning commands, from a user and to output sensory perceptible output to a user. In addition, an actuator is provided for actuating the movable arm part to effect the longitudinal movement of the surgical instrument. Another term for actuator is driving mechanism. Additionally, a sensor is provided for obtaining sensor data indicative of a distance between the surgical instrument, e.g. the tip of the surgical instrument, and the surgical target.

A processor subsystem is provided for controlling the actuator to actuate the movable arm part to move the surgical instrument along the longitudinal axis. At an initial position of the surgical instrument, the processor subsystem may obtain sensor data from the sensor and determine an initial distance between the surgical instrument and the surgical target. The processor subsystem may obtain data indicating a target position of the surgical instrument relative to the surgical target. For example, data indicating the target position may be obtained from a user in the form of a user input, or may be automatically determined based on the detection of particular anatomical structures, or the like. The target position may be the position of the surgical target, or another position, for example between the surgical target and the initial position of the surgical instrument. The processor subsystem may determine a displacement distance for the surgical instrument based on the sensor data and the target position. For example, the displacement distance may be a distance through which the surgical instrument will be moved in a single first movement. The processor subsystem may output a sensory-perceptible representation of the determined displacement. The sensory-perceptible representation may be, for example, an audio output indicating the determined displacement distance, an acoustic output, e.g. indicating a proximity, a graphical output, such as an overlay on an image of the interior of an eye with the determined displacement distance labelled, or a textual output, such as a visual output of a number representing the determined displacement distance, a haptic feedback, and/or the like. The user may then confirm the determined displacement distance, and the processor subsystem may receive a confirmation signal via the user interface. The confirmation signal may be any kind of user input indicating that the user confirms the determined displacement distance, including a voice command, a click of a button, for example on a graphical user interface, or the like. Once the confirmation signal has been received, the processor subsystem may control the actuator to actuate the movable arm part to effect a single movement of the surgical instrument along the longitudinal axis over the determined displacement distance. That is, the user confirms a distance through which the surgical instrument is moved. Here, the term 'single movement' may also be considered to be a single fluid movement, without breaks or pauses interrupting the movement. The term 'longitudinal movement towards or away from the surgical target' refers to an advancing movement of the surgical instrument or a retracting movement of the surgical instrument.

By determining a displacement distance for the surgical instrument, the user (e.g. surgeon) is able to retain control over the placement and progression of a surgical instrument when performing surgery in or on the eye, whilst performing the movement in an automated fashion. Since the measurements are based on sensor data, there is a risk that the sensor data is too noisy to provide a reliable measurement. This can lead to unreasonable distances being calculated. Having the user approve a determined displacement distance, before the single movement of the surgical instrument is carried out, thus provides the user an opportunity to prevent movement of the surgical instrument when the sensor data is unreasonable or inaccurate. That is, the user is required to confirm the next step of the surgical instrument's movement before said movement is carried out. The determined displacement distance is communicated to the user, and the user is requested to confirm the determined displacement distance. By doing so, if the determined displacement distance is unreasonable, then the user can disapprove the determined displacement distance. This allows the user to retain control over the procedure, whilst the movement of the surgical instrument itself is carried out in an automated way, reducing or eliminating risks associated with a manual injection, such as hand tremors or shaking. Moreover, the displacement distance over which the surgical instrument is to be moved in a single movement is determined after the sensor data indicating the distance measurement is obtained, and the sensor data is preferably obtained whilst the surgical instrument is stationary. This may increase the reliability of the sensor measurements, particularly if the sensor data is obtained whilst the surgical instrument is in a static position.

Optionally, the processor subsystem may, upon completing the single movement of the surgical instrument, obtain updated sensor data from the sensor and determine a remaining distance between the surgical instrument and the surgical target based on the updated sensor data, determine whether the target position is reached based on the determined remaining distance, and, if not, control the actuator to correct a depth of the surgical instrument based on the determined remaining distance. Any movement of the eye or the structures within the eye, for example due to changes in intraocular pressure, breathing, patient movement or a reaction to the movement of the surgical instrument, which may have shifted the target position and/or the surgical target, can thus be taken into consideration. Moreover, updated sensor data may provide a more reliable measurement, such as if an initial measurement was partially or fully obscured, e.g. by debris or other structures or tissues in or around the eye, or the like. The updated sensor data may be obtained whilst the surgical instrument is in a different position, likely closer to the surgical target, which may be less noisy.

Optionally, the processor subsystem may control the actuator in a displacement-based control mode to correct the depth of the surgical instrument by determining a correction displacement distance to reach the target position from the determined remaining distance to the surgical target, outputting, via the user interface, a sensory-perceptible representation of the correction displacement distance, receiving, via the user interface, a further confirmation signal from the user, and upon receiving the further confirmation signal, controlling the actuator to actuate the movable arm part to effect a further single movement of the surgical instrument along the longitudinal axis over the correction displacement distance. In this correction procedure, updated sensor data is obtained before the surgical instrument is moved to correct its position. The correction displacement distance is then determined, after the updated sensor data is obtained. Since the user is once again asked to confirm the determined correction displacement distance, they are provided another opportunity to ensure that the distance through which the surgical instrument will be moved is reasonable. This therefore improves the safety and reliability of the further step of movement of the surgical instrument.

Optionally, the processor subsystem may control the actuator in a limited-closed-loop control mode in which updated sensor data is used to correct the depth of the surgical instrument, wherein the limited-closed-loop control mode is limited by at least one of a predefined time duration for use of the limited-closed-loop control mode, a predefined maximum correction displacement distance, requiring a certainty score to remain above a certainty score threshold during the correction, the certainty score representing a certainty of the determined remaining distance, and requiring a continued user input during the correction and stopping movement of the surgical instrument upon detecting that the continued user input has ended. The level of automation of the system may thus be controlled in this mode by the factors described above, thereby preventing a fully automated system which may not be readily adopted by users, whilst still providing the advantages in movement of automated processes. For example, the requirement of a certainty score being above a threshold ensures that the movement of the surgical instrument may be automated when the sensor data is sufficiently reliable, and prevents automated movement whilst the sensor data is unreliable, which may lead to overshooting or undershooting a target position. Requiring a continued user input during the correction may ensure that the user is involved and paying attention. Time or displacement limitations may similarly limit the extent to which the surgical robotic system is automated. For example, the automated movement of the surgical instrument may stop after some amount of time or after some displacement and then, for example, may resume with updated sensor data. This limited level of automation of the system may therefore improve patient safety during the surgical procedure.

Optionally, the processor subsystem may calculate a certainty score representing a certainty of a determined distance between the surgical instrument and the surgical target, and adapt the control of the actuator according to the calculated certainty score. The certainty score may be based on at least one of an estimate of measurement noise of the sensor, a detection of a presence of a dynamic movement of the surgical target, a comparison of the sensor data with a model representing a reference for the sensor data, and a comparison between an estimated surgical target position and a predicted surgical target position, wherein the predicted surgical target position is based on instrument movements as measured by at least one position sensor. For example, if the certainty score is high, the speed of the surgical instrument may be increased, whereas if the certainty score is low, the speed of the surgical instrument may be decreased. In this way, the procedure may proceed quicker when the sensor data is more reliable (e.g. high certainty score), thereby reducing the duration (and therefore prolonged discomfort of the patient) of the surgical procedure and the surgical instrument may move slower when the sensor data is less reliable, preventing the surgical instrument from extending beyond the surgical target. The use of a certainty score and the adaptation of the control of the actuator according to the certainty score may offer enable a user (e.g. surgeon) to determine when more manual control is required to ensure patient safety and surgical precision, and when it is possible to make use of a more automated approach, which may make the procedure quicker, reducing patient discomfort.

Optionally, the processor subsystem may calculate the certainty score repeatedly and may perform an abort procedure if the calculated certainty score falls below a certainty score threshold. The abort procedure may comprise at least one of retracting the surgical instrument, pausing movement of the surgical instrument, and switching from controlling the actuator in the limited-closed-loop control mode to controlling the actuator in a motion-controller-based control model, in which the processor subsystem may control the actuator according to position control commands received from the user via a motion controller. Calculating a certainty score repeatedly may prevent proceeding with movement of the surgical instrument if the sensor data is too noisy or unreliable, thereby reducing the risk of unintentional movement of the surgical instrument and damage to the structures within or on the eye.

Optionally, the processor subsystem may select a control mode from a set of control modes based on the calculated certainty score, the set of control modes comprising at least two of a motion-controller-based control mode, a displacement-based control mode and a limited-closed-loop control mode. In the motion-controller-based control mode, the processor subsystem may control the actuator according to position control commands received from the user via a motion controller of the user interface. In the displacement-based control mode, the processor subsystem is configured to determine a correction displacement distance to reach the target position from the determined remaining distance to the surgical target, output, via the user interface, a sensory-perceptible representation of the correction displacement distance, receive, via the user interface, a further confirmation signal from the user, and upon receiving the further confirmation signal, control the actuator to actuate the movable arm part to effect a further single movement of the surgical instrument along the longitudinal axis over the correction displacement distance. In the limited-closed-loop control mode, the processor subsystem may be limited by at least one of a predefined time duration for use of the limited-closed-loop control mode, a predefined maximum correction displacement distance, requiring a certainty score to remain above a certainty score threshold during the correction, the certainty score representing a certainty of the determined remaining distance, and requiring a continued user input during the correction, and stopping movement of the surgical instrument upon detecting that the continued user input has ended. Using the certainty score to select a control mode may ensure that more user input is involved, for example when the certainty score is low, whilst allowing a greater degree of automation in some cases, e.g. when the certainty score is high.

Optionally, the processor subsystem may output, via the user interface, a sensory-perceptible representation of the calculated certainty score, prior to receiving the confirmation signal from the user. Providing a sensory-perceptible representation of the certainty score to the user indicates the level of certainty associated with the sensor measurement, allowing the user to either confirm or disallow the progression of the procedure. For example if the certainty score indicates a low degree of certainty, the user may decide not to proceed with the single movement of the surgical instrument, and/or may decide to update sensor data again or the like.

Optionally, the sensor comprises at least one of an optical coherence tomography (OCT) sensor configured to optically couple to an optical fiber attached to or integrated in the surgical instrument, intraoperative optical coherence tomography (iOCT) through a microscope, a stereo camera through a microscope, an optical interferometric sensor integrated in the surgical instrument, a time-of-flight sensor integrated in the surgical instrument, and an ultrasonic sensor integrated in the surgical instrument.

Optionally, the processor subsystem is further configured to obtain sensor data indicative of a distance between a first layer of tissue of an eye and a second layer of tissue of the eye, wherein the surgical target is between the first layer and the second layer, and correct the position of the surgical instrument such that the surgical instrument is between the first layer and the second layer. By determining the distance from the surgical instrument and each of the first and second layers respectively, the system may determine whether the surgical instrument is between the two layers. This can improve the positioning of the surgical instrument.

Optionally, the processor subsystem may obtain sensor data indicative of a distance between a first layer of tissue of an eye and a second layer of tissue of the eye, wherein the surgical target is between the first layer and the second layer, and wherein the data indicative of a target position of the surgical instrument relative to the surgical target comprises data indicative of the distance between the first layer and the second layer. Determining the distance between layers of tissue may improve accuracy of the positioning of the surgical instrument.

It will be appreciated by those skilled in the art that two or more of the above-mentioned embodiments, implementations, and/or aspects of the invention may be combined in any way deemed useful.

Modifications and variations of the method and/or the computer program product, which correspond to the described modifications and variations of the surgical robotic system, can be carried out by a person skilled in the art on the basis of the present description.

The invention is defined in the independent claims or clauses. Advantageous yet optional embodiments are defined in the dependent claims or clauses.

### BRIEF DESCRIPTION OF THE DRAWINGS

These and other aspects of the invention are apparent from and will be elucidated with reference to the embodiments described hereinafter. In the drawings,
Fig. 1 shows a schematic representation of a surgical robotic system;
Fig. 2 shows a surgical instrument passing through a trocar during minimally invasive surgery, the surgical instrument having four degrees of freedom (DoFs);
Fig. 3 shows a joint diagram illustrating the kinematics of a movable arm part of a surgical arm for use in minimally invasive surgery;
Fig. 4 shows a joint diagram illustrating the kinematics of a motion controller;
Fig. 5 shows a surgical instrument in an eye according to an embodiment of the invention;
Fig. 6A shows a surgical instrument at an initial position in an eye according to an embodiment of the invention;
Fig. 6B shows a surgical instrument after a single movement of the surgical instrument has been effected according to an embodiment of the invention;
Fig. 7A schematically shows a method of selecting a control mode according to an embodiment of the invention;
Fig. 7B schematically shows a method for selecting a control mode using a certainty score, according to an embodiment of the invention;
Fig. 8A illustrates thresholds for selecting a control mode according to an embodiment of the invention;
Fig. 8B illustrates thresholds for selecting a control mode and/or triggering an abort procedure according to an embodiment of the invention;
Fig. 9 schematically shows a method for triggering an abort procedure according to an embodiment of the invention;
Fig. 10A shows a surgical target between two layers of tissue prior to an injection, according to an embodiment of the invention;
Fig. 10B shows an indented retina prior to an injection, according to an embodiment of the invention;
Fig. 10C shows a surgical target between two layers of tissue during an injection, according to an embodiment of the invention;
Fig. 11 schematically shows a method for controlling a surgical robotic system during use in an ocular surgical procedure; and
Fig. 12 shows a computer program product comprising instructions for causing a processor system to perform the method.

It should be noted that items which have the same reference numbers in different figures, have the same structural features and the same functions, or are the same signals. Where the function and/or structure of such an item has been explained, there is no necessity for repeated explanation thereof in the detailed description.

### List of reference numerals

The following list of reference numerals is provided for aiding the interpretation of the drawings and shall not be construed as limiting the claims or clauses.
020 User interface
022 User inputs
030 Sensor
032 Sensor data
040 Processor subsystem
042 Actuation commands
060 Actuator
062 Actuation of surgical arm
080 Surgical arm
082 Movable arm part
100 Surgical robotic system
101 *̅e̅*̅₁, axis of a Cartesian coordinate system
102 *e̅_{y},* axis of a Cartesian coordinate system
103 *e̅_{z}*, axis of a Cartesian coordinate system
104 *e̅ₓ*, axis of coordinate system fixed to the instrument tip, orthogonal to the instrument longitudinal axis
105 *e̅_{y}*, axis of coordinate system fixed to the instrument tip, orthogonal to the instrument longitudinal axis
106 *e̅_{z}*, axis of a Cartesian coordinate system, aligned with the instrument longitudinal axis
107 *φ*, rotation of surgical instrument, laterally displacing its tip
108 *ψ*, rotation of surgical instrument, laterally displacing its tip
109 *z*, longitudinal (along its longitudinal axis) translation of surgical instrument, or penetration direction, or advancing direction
110 θ, rotation of surgical instrument around its longitudinal axis
111 *Φ*, rotational DoF of a movable arm part
112 *ψ*, rotational DoF of a movable arm part
113 Z, translational DoF of a movable arm part
114 *Θ*, rotational DoF of a movable arm part
115 *Φₘ*, rotational DoF of motion controller
116 *ψₘ*, rotational DoF of motion controller
117 *Zₘ*, translational DoF of motion controller
118 *Θₘ*, rotational DoF of motion controller
119 Surgical instrument
121 Distance sensor
122 Surgical instrument tip
123 Surgical target
124 Trocar
125 Remote center of motion (RCM)
126 Button on motion controller gripper
127 Optical beam
200 Eye
210 Retina
220 First layer of tissue
230 Second layer of tissue
240 Bleb
700 A method for selecting a control mode
710 Calculating a certainty score
720 Comparing certainty score to first threshold
730 Decision block
740 Selecting motion-controller-based control mode
744 Comparing certainty score to second threshold
746 Decision block
750 Selecting displacement-based control mode
760 Selecting limited-closed-loop control mode
810 Motion-controller-based control mode
820 Displacement-based control mode
830 Limited-closed-loop control mode
840 Abort procedure
900 A method for triggering an abort procedure
910 Calculating a certainty score
920 Comparing certainty score to threshold
940a Continuing movement of the surgical instrument
940b Continuing with ocular surgical procedure
950 Performing abort procedure
1100 A method for controlling a surgical robotic system
1110 Obtaining sensor data
1120 Determining an initial distance
1130 Obtaining target position with respect to surgical target
1140 Determining displacement distance
1150 Outputting displacement distance
1160 Receiving user confirmation
1170 Actuating movable arm to move in the single movement
1150 Non-transitory program code
1160 Computer readable medium

### DETAILED DESCRIPTION OF EMBODIMENTS

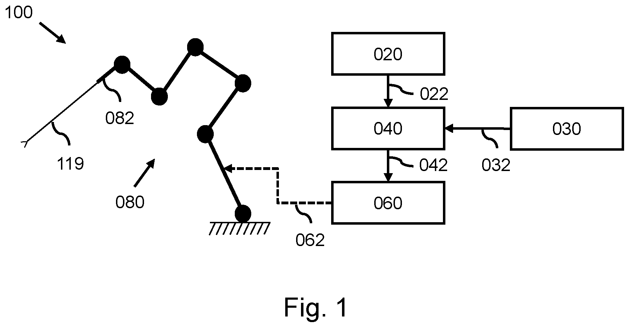

**Fig. 1** schematically shows a surgical robotic system 100 for use in a surgical procedure. The surgical robotic system 100 comprises a surgical arm 080. The surgical arm 080 comprises a movable arm part 082, with the movable arm part comprising an instrument connector for mounting of a surgical instrument 119. Fig. 1 shows the surgical instrument 119 having been mounted to the instrument connector (for sake of simplicity, the instrument connector is not separately shown in Fig. 1). The movable arm part 082 has at least one degree-of-freedom (DoF) to enable longitudinal movement of the surgical instrument towards a surgical target. Here, longitudinal movement refers to a movement of the surgical instrument 119 along its longitudinal axis (for sake of simplicity, the longitudinal axis is not separately shown in Fig. 1).

The surgical robotic system 100 further comprises a user interface 020 for receiving inputs from a user. The user inputs 022 may comprise positioning commands from a human operator, e.g. a surgeon or healthcare provider, for controlling the longitudinal movement of the surgical instrument, a confirmation input indicating instructions to continue with a procedure or procedural step, and/or input information, such as indicating an operating parameter and/or an indication of a surgical target position or the like. Examples of user interfaces include, but are not limited to, a keyboard, a mouse, a touch-sensitive surface, a joystick, a foot pedal, a microphone, a gesture recognition system and/or the like. The user interface may employ any suitable input modality, such as touch, push-actions, voice commands, eye movements, gesture recognition, etc.

The surgical robotic system 100 further comprises an actuator 060 configured and arranged for actuating the movable arm part to effect the longitudinal movement of the surgical instrument. The actuator 060 may be any suitable actuator, e.g., from the field of surgical robots, or from the more general field of actuators. In particular, the actuator may be one of a plurality of actuators which together provide the actuation of the movable arm part 060 along the at least one DoF. Namely, the surgical robotic system 100 may comprise a plurality of actuators, e.g., to provide actuation along multiple DoF. As such, it will be appreciated that any reference to a configuration of the actuator 080 may be understood as referring to a (joint) configuration of such a plurality of actuators. Fig. 1 shows the actuation of surgical arm 080 schematically, namely as a dashed line 062. It is noted that, although shown separately of the surgical arm 080, the actuator 060 may be integrated into, or mounted to, the surgical arm 080.

The surgical robotic system 100 further comprises a sensor 030 configured and arranged for obtaining sensor data 032 indicating a distance between the surgical instrument 119 and the surgical target. The sensor 030 may comprise at least one of an optical coherence tomography (OCT) sensor configured to optically couple to an optical fiber attached to or integrated in the surgical instrument 119, intraoperative optical coherence tomography (iOCT) through a microscope, a stereo camera through a microscope, an optical interferometric sensor integrated in or attached to the surgical instrument 119, a time-of-flight sensor integrated in or attached to the surgical instrument 119 and an ultrasonic sensor integrated in or attached to the surgical instrument 119. The surgical instrument may be an irrigation/aspiration instrument for irrigating or aspirating fluid at a desired target location, a photocoagulation instrument for applying optical energy at a desired distance from a target, a vitrectomy instrument for cutting and aspirating the vitreous humor or other fluidics at a desired distance from a target, a tissue manipulating instrument, which needs to be placed exactly at the first interface of a tissue, or the like. In some embodiments, the surgical robotic system 100 may comprise a microscope, e.g. configured to provide a stereo camera and/or iOCT. In some embodiments, a microscope may be communicatively coupled to the surgical robotic system 100. In some embodiments, the sensor data 032 may comprise A-scans, comprising line measurements and defined as an intensity as a function of the distance, B-scans forming a 2D image, C-scans, e.g. from multiple B-scans, or the like. Distances may be derived from any of these scan types using, for example, image analysis. In some embodiments, the sensor 030 may comprise an optical sensor and the sensor data 032 may comprise diffuse reflectance. In some embodiments, the sensor 030 may comprise a stereo camera and the sensor data 032 may comprise two 2-D images to obtain a depth map.

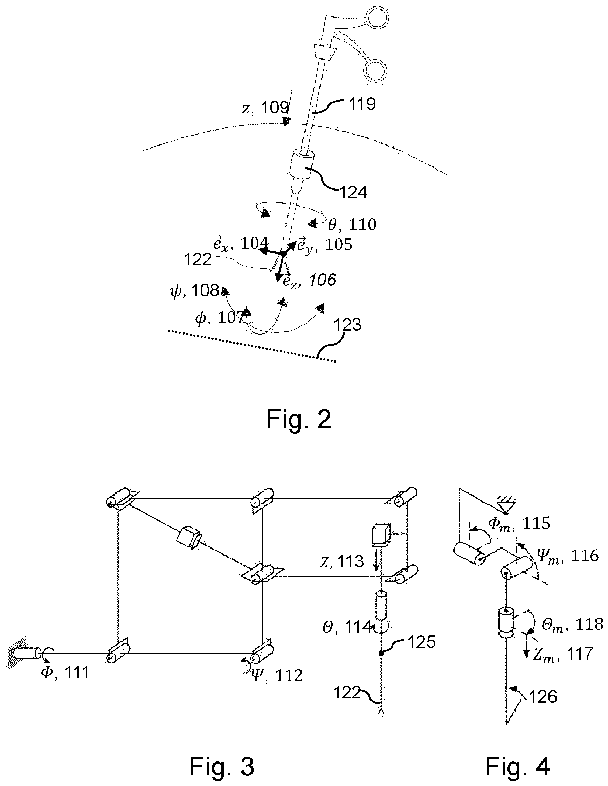

The surgical robotic system 100 further comprises a processor subsystem 040 configured for controlling the actuator to actuate the movable arm part, moving the surgical instrument 119 along the longitudinal axis. When the surgical instrument 119 is at an initial position, processor subsystem 040 may obtain sensor data 032 from the sensor 030. The sensor data 032 may be indicative of a distance between the surgical instrument 119, e.g. the tip 122 of the surgical instrument 119 (illustrated in Fig. 2), and the surgical target 123 (illustrated in Fig. 2). In other words, the sensor data 032 may be used to determine the distance between the surgical instrument and the surgical target. In some embodiments, the sensor data 032 is obtained whilst the surgical instrument is stationary, in order to improve the reliability of the measurement. In some embodiments, the sensor 030 may be configured to obtain sensor data 032 indicating a distance between multiple layers of tissue of an eye. That is, the sensor 030 may be configured to determine a distance between the surgical instrument and a first layer of tissue, and a distance between the surgical instrument and a second layer of tissue.

The processor subsystem 040 may be configured to obtain data indicative of a target position of the surgical instrument relative to the surgical target. The position of the surgical target itself may be identified from sensor data, e.g., from an internal or external sensor, but may alternatively or additionally also be specified by user input. The data indicative of the target position may typically be obtained from user input, but may alternatively or additionally also be determined automatically. In general, the target position may be a relative position which is relative to the surgical target. In some embodiments, the target position may be defined as a one-dimensional position on a line from the surgical instrument to the surgical target. That is, the target position may be defined as a longitudinal position. Such a target position may elsewhere also be referred to as a 'depth', for example if the target position is longitudinally beyond the surgical target, or as a 'proximity', for example if the target position is longitudinally before the surgical target. In some embodiments, the target position may be defined as a specified distance from the surgical target. Whether the target position is beyond or before the surgical target may be indicated by a sign of the specified distance. The target position may also have any other dimensionality, for example a three-dimensional position in a polar or cartesian coordinate system. In some embodiments, the user may be requested to input the target position graphically, e.g. by providing an input on a graphical user interface, from which graphical input the processor subsystem 040 may then determine the target position numerically. In other embodiments, the user may be requested to input the target position directly in numerical form, e.g., by providing a numerical input indicating a distance relative to the surgical target. In some embodiments, where the surgical target is a layer of tissue, the target position may be provided as a relative position or depth from the layer of tissue. The layer of tissue may for example be a layer of vitreous tissue or a layer of retinal tissue.

The processor subsystem 040 may be configured to determine a displacement distance for the surgical instrument based on the sensor data and the target position. For example, the displacement distance may be a function of the difference between the initial position and the target position. In some embodiments, the displacement distance may be a partial distance between the initial position of the surgical instrument and the target position. In other embodiments, the displacement distance may be the total distance between the initial position of the surgical instrument and the target position.

The processor subsystem 040 may be configured to output, via the user interface 020, a sensory-perceptible representation of the determined displacement distance. The sensory-perceptible representation of the determined displacement distance may comprise a graphical representation of the determined displacement distance, such as visually overlaid on an image of the surgical field, an audio output, e.g. via a speaker of the user interface 020, and/or a visual numerical output, e.g. on a display of the user interface 020. The sensory-perceptible representation of the determined displacement distance may be rounded to specified level of precision.

The processor subsystem 040 may be configured to receive, via the user interface, a confirmation signal from the user. The confirmation signal may be received as an audio input, e.g. via a microphone of the user interface 020, a touch input or click input, e.g. on a graphical user interface of the user interface 020, an input into a motion controller, a foot pedal input, a gesture-based input, e.g. via a camera of the user interface 020, and/or the like. The user interface 020 may be configured to receive one or more types of input for the confirmation signal.

Upon receiving the confirmation signal, the processor subsystem 040 may be configured to control the actuator to actuate the movable arm part to effect a single movement of the surgical instrument along the longitudinal axis over the determined displacement distance. That is, once the user has confirmed the determined displacement distance, the surgical instrument is moved over the determined displacement distance in a single, uninterrupted movement. The single movement of the surgical instrument may be referred to as a continuous movement or an uninterrupted movement. In some embodiments, the surgical instrument may only be moved toward the surgical target if the user confirms the determined displacement distance.

In some embodiments, the surgical robotic system 100 may be configured to perform a correction procedure. Upon completing the single movement of the surgical instrument, the processor subsystem 040 may be configured to obtain updated sensor data 032 from the sensor 030 and determine a remaining distance between the surgical instrument and the surgical target, based on the updated sensor data 030. In other words, after the surgical instrument has been moved over the determined displacement distance to a second position, updated sensor data is obtained whilst the surgical instrument is in the second position. In some embodiments, the updated sensor data is obtained whilst the surgical instrument is stationary, in order to improve the reliability of the measurement. The processor subsystem 040 may be configured to determine whether the target position is reached based on the determined remaining distance. If the processor subsystem 040 determines that the target position has not been reached by the surgical instrument, the processor subsystem 040 may control the actuator to correct a depth of the surgical instrument based on the determined remaining distance. In some cases, movement of the surgical instrument and/or the eye, e.g. changing intraocular pressure or patient movement, may displace the target position. In some cases, the initial sensor measurement may have been slightly inaccurate, leading to an inaccurate displacement distance, and ultimately resulting in a movement of the surgical instrument that is either too short or too long, undershooting or overshooting the target position. In such cases, the depth, e.g. the position, of the surgical instrument may be corrected based on the determined remaining distance.

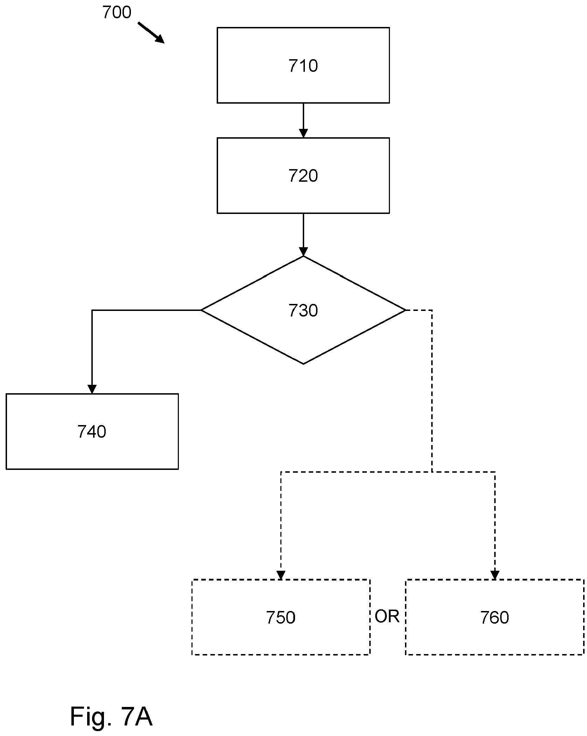

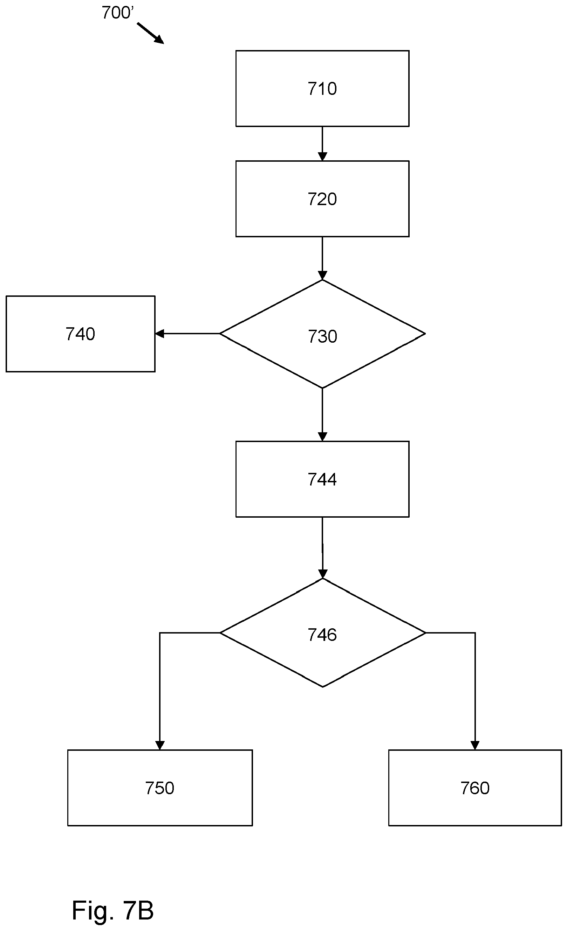

The processor subsystem 040 may control the actuator in one or more control modes, which will be described in detail with reference to Figs. 7-9, for example based on a certainty score. In some embodiments, a control mode, such as those described with reference to Figs. 7-9, may be manually selected.

**Fig. 2** shows a surgical instrument 119 passing through a trocar 124 during minimally invasive surgery. For example, in case of vitreoretinal surgery, the trocar 124 may be placed in the sclera. Rotating around and translating through the trocar may be possible in four DoF, e.g., the rotations *φ* 107, *ψ* 108, *θ* 110 and the translation z 109 to approach or penetrate a surgical target 123. Further shown are a tip 122 of the surgical instrument 119 and three axes 104-106 of a coordinate system fixed to the instrument tip 122, with *e̅_{z}* 106 aligned with the longitudinal axis of the surgical instrument 119. Rotations *φ* 107 and *ψ* 108 may result in a lateral displacement of the instrument tip 122, respectively in the direction *e̅_{y}* 105 and in the direction *e̅ₓ* 104. The translation *z* 109 may result in a longitudinal movement of the surgical instrument tip 122.

The surgical robotic system 100 may be used in an ocular surgical procedure, such as a minimally invasive surgical procedure as described above. **Fig. 3** shows a joint diagram illustrating the kinematics of a movable arm part of a surgical arm for use in an ocular surgical procedure, and in particular a minimally invasive surgery. In the example of Fig. 3, the surgical robotic system comprises a surgical arm, with the surgical arm comprising a movable arm part having DoFs *Φ* 111, *ψ* 112, *Z* 113 and *Θ* 114, allowing respective instrument motions 107-110, resulting in movements of the surgical instrument tip 122. The DoFs may be arranged such that there is a point on the surgical instrument that does not move in space, termed the Remote Center of Motion (RCM) 125. By moving the base of the surgical arm, the movable arm part may be positioned, such that its RCM 125 may be positioned at the trocar. Respective actuation units may be arranged to effect movement in all four DoFs 111-114.

The surgical robotic system may further comprise a user interface for receiving user inputs, such as positioning commands, from a human operator, e.g. a surgeon or healthcare provider. In some embodiments, the user interface may comprise or be constituted by a motion controller such as a joystick. In some embodiments, the motion controller may be an external device with which the user interface or the processor subsystem 040 is configured to interface. In some embodiments, the motion controller may be comprised in the surgical robotic system as a further component, e.g. configured to interface with the processor subsystem 040 and/or the user interface 020. **Fig. 4** shows a joint diagram illustrating the kinematics of such a motion controller. Here, the motion controller is shown to have DoFs *Φₘ* 115, *ψₘ* 116, *Zₘ* 117 and *Θₘ* 118. The user may provide user inputs, such as positioning commands by, e.g., holding the motion controller at a gripper part, pressing the button 126, and moving the gripper part of the motion controller in 3D space.

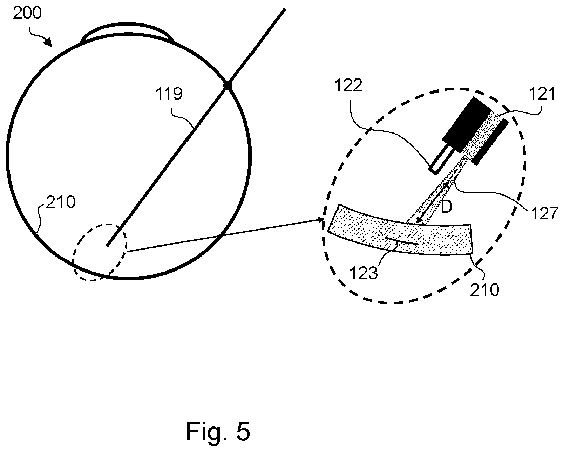

**Fig. 5** shows a surgical instrument 119 in an eye 200 according to an embodiment of the invention. The tip 122 of the surgical instrument 119 is also shown magnified.

As illustrated on the left hand side of Fig. 5, the surgical instrument 119 is shown inserted into the eye 200. The surgical instrument 119 may be approaching the retina 210 as illustrated in this figure, although this is merely exemplary and the invention is not limited thereto. In some embodiments, the surgical instrument 119 may approach another area, structure or tissue layer of the eye.

The tip 122 of the surgical instrument 119 is illustrated on the right hand side of Fig. 5, magnified. In this example, the surgical target 123 is located in the retina. The surgical instrument may be an irrigation/aspiration instrument for irrigating or aspirating fluid at a desired target location, a photocoagulation instrument for applying optical energy at a desired distance from a target, a vitrectomy instrument for cutting and aspirating the vitreous humor or other fluidics at a desired distance from a target, a tissue manipulating instrument, which needs to be placed exactly at the first interface of a tissue, or the like. The tip 122 of the surgical instrument 119 may be an injection lumen. A sensor or sensor component 121, such as an optical fiber, may be integrated in or attached to the surgical instrument 119. In some embodiments, the sensor 030 may comprise an optical fiber (as shown), configured to obtain sensor data 032 indicative of a distance D between the surface of the retina 210 and the tip 122 of the surgical instrument 119. The optical fiber 121 may be recessed from the tip 122 of the surgical instrument 119. In some embodiments, the sensor 030 may be configured to determine the distance between the end of the optical fiber 121 and the surface of the retina 210, and then subtract an offset corresponding to the distance through which the optical fiber 121 is recessed from the tip 122 of the surgical instrument 119, to obtain distance D. In some embodiments, the distance D may be determined using an optical beam 127. Since the optical fiber 121 is recessed from the tip 122 of the surgical instrument 119, it is possible and advantageous to obtain sensor data whilst the tip 122 of the surgical instrument 119 is piercing or penetrating a tissue layer.

The distance D between the surface of the retina 210 and the tip 122 of the surgical instrument 119 may be used to determine the distance between the tip 122 of the surgical instrument 119 and the surgical target 123. For example, the surgical target 123 may be a layer of the retina 210, or a surface thereof, at a known depth, and the distance between the tip of the surgical instrument and the surgical target 123 may be determined by adding the known depth of the surgical target 123 to the distance D, between the tip 122 of the surgical instrument 119 and the surface of the retina 210.

In some embodiments, the target position may correspond to the surface of the retina 210. More generally, the target position may be determined based on the position or detection of a structure or tissue layer, such as the surface of the retina 210.

In the foregoing example, reference has been primarily made to the distance between the tip 122 of the surgical instrument 119 and, for example, the surface of the retina 210. It is to be understood, however, that the invention is not limited thereto. In some embodiments, the distance may be measured from another part of the surgical instrument 119, instead of or in addition to the tip 122. For example, distances may be determined with respect to the position of the optical fiber, a marker on the surgical instrument 119 or the like.

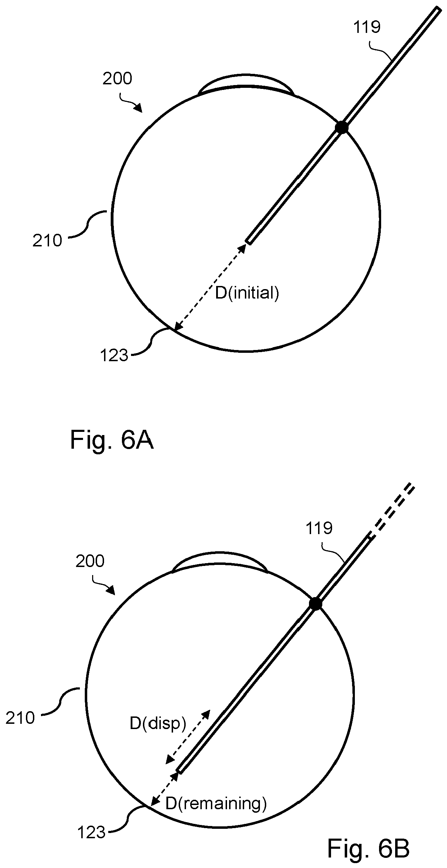

**Fig. 6A** shows a surgical instrument 119 at an initial position in an eye 200 according to an embodiment of the invention. As in Fig. 5, the surgical instrument 119 is shown approaching a point along the surface of the retina 210. It is reiterated here that this choice of surgical target is merely exemplary - the methods and systems disclosed herein apply analogously regardless of the choice of surgical target. In Fig. 6A, the surgical instrument 119 is shown in an initial position, a distance D(initial) from the surgical target 123. Whilst in the initial position, the distance D(initial) may be determined, for example using a sensor 030 coupled to, attached to or integrated in the surgical instrument 119, as shown in Fig. 5. Preferably, the sensor measurement is performed whilst the surgical instrument 119 is stationary. Once the distance between the surgical instrument 119, e.g. the tip 122 of the surgical instrument 119, and the surgical target 123 is known, a displacement distance may be determined. The displacement distance is a distance through which the surgical instrument 119 is configured to move, and may be defined as a distance between the surgical instrument 119 and a target position. The displacement distance may be the (e.g. total) distance to the surgical target 123, or the displacement distance may be shorter than the distance to the surgical target 123. For example, if the surgical target 123 is located in a particularly complicated and/or delicate part of the eye 200, or if the location of the surgical target 123 is difficult to read using sensors, then it may be advantageous to first move the surgical instrument 119 a first distance, to a next position, in which more reliable sensor data may be obtained. Selecting a displacement distance shorter than the initial distance between the surgical instrument 119 and the surgical target 123 may further be advantageous if movement of the eye 200 or the patient during movement of the instrument is likely, e.g. if the structures within the eye 200 and/or the surgical target 123 are likely to move as a consequence of the movement of the surgical instrument.

The displacement distance may be determined by obtaining a user input indicative of a target position of the surgical instrument, such an input specifying a target depth or even indicating a specific target position, for example on a graphical user interface. In some embodiments, the target position may correspond to the surgical target, and the user may simply indicate that the target position is the position of the surgical target. In some embodiments, the displacement distance may be determined using a predetermined setting, such as a defined offset from the surgical target. Such an offset could be, for example, 2mm short of the surgical target. In some embodiments, the displacement distance may be automatically determined, for example based on the detection of one or more anatomical structures, tissue layers or the like, or based on the noise in the sensor data. For example, if the sensor data is noisy, a shorter displacement distance may be determined. In some embodiments, the displacement distance may be automatically determined, for example based on the initial distance, such as to cover a predetermined fraction of the initial distance.

Once the displacement distance has been determined, the surgical robotic system 100 may be configured to output a sensory-perceptible representation of the determined displacement distance. The user is requested to confirm the determined displacement distance, before the surgical instrument 119 may be moved. Requiring user confirmation of the determined displacement distance enables the user to ensure that the value determined by the system 100 is reasonable - as inaccurate or noisy sensor data or detection may lead to an inaccurate displacement distance being calculated, risks associated with moving the surgical instrument through an unsuitable (e.g. too far, or unnecessarily short) distance may be mitigated. This step also provides the user to be directly involved with the movement of the surgical instrument 119, dispelling some of the discomfort typically associated with automation in this field, without introducing risks associated with hand tremors, shaking and similar.

User confirmation may be provided by means of, for example, the click of a button, such as in a graphical user interface, on a mouse or motion controller, a press of a foot pedal, a verbal acknowledgement using a microphone and speech recognition, gesture recognition of a gesture captured using a camera configured for gesture capture, or any combination thereof, or the like. Any or all of these means of user interaction may be incorporated in, or used via, the user interface.

Upon obtaining a user confirmation signal, the surgical instrument 119 may be moved through the determined displacement distance, in a single movement. That is, the movement of the surgical instrument 119 through the determined displacement distance may be free of pauses or interruptions. The new position of the surgical instrument 119 is shown in Fig. 6B. In some embodiments, the movement of the surgical instrument 119 through the determined displacement distance may be carried out at a constant velocity or with a constant acceleration, or the velocity or acceleration of the surgical instrument 119 may be nonlinear.

In some embodiments, aspects of the movement of the surgical instrument 119, such as speed, acceleration, or the like, may be adapted based on a certainty score. The certainty score may represent a certainty of a determined distance between the surgical instrument and the surgical target and/or a certainty of the obtained sensor data 032. The certainty score may also be referred to as a confidence score, representing the confidence of a determined distance and/or sensor data 032, or a reliability score, indicating the reliability of a measurement or calculation, e.g. of a determined distance or of obtained sensor data. In other words, the certainty score is a quantifiable measure of the correctness and reliability of a measurement. That is, a certainty score may represent the certainty of the initial distance (e.g. before the surgical instrument 119 has been moved) between the surgical instrument 119 and the surgical target, or the certainty of the determined displacement distance between the surgical instrument 119 (e.g. before the surgical instrument 119 has been moved) and the target position. For example, the certainty score may be low due to measurement noise, measurement artifacts, and/or the sensor being at least partly blocked, e.g. by debris in the eye. The certainty score may be calculated based on at least one of the noise associated with the obtained sensor data, movement of the surgical target, a comparison between the sensor data and a reference model, and a comparison between an estimated surgical target position and a predicted position of the surgical target.

The predicted surgical target position may be based on instrument movements in robotic coordinates as measured by at least one robotic position sensor. In some embodiments, the robot 080 may comprise a suitable position sensor, and may be used determine the instrument movements.

Any known statistical method for determining a certainty may be used in the calculation of a certainty score based on one or more of the above considerations. It may also be an output of template matching algorithms, Kalman filters, Bayesian filters and / or machine learning algorithms.

When the certainty score is high, the surgical instrument 119 may be controlled to move at a faster speed than when the certainty score is low, for example. Moreover, if an initial certainty score is calculated, the target position may be, at least partially, determined based on the certainty score. For example, if the initial certainty score is low, e.g. below a threshold, then the displacement distance may be shorter than if the initial certainty score were high (e.g. above a threshold). The certainty score may also be used to select a control mode, as elucidated further with reference to Fig. 6B.

In some embodiments, a certainty score may be calculated prior to the single movement of the surgical instrument 119. That is, a sensory-perceptible representation of a certainty score may be output to the user, as well as a sensory-perceptible representation of the determined displacement distance, prior to the user providing a confirmation signal. In this way, the user may confirm or not confirm the determined displacement distance whilst also taking an associated certainty score into consideration. In some embodiments, the determined displacement may be limited as a result of the certainty score - for example, if the certainty score is low, e.g. lower than a threshold, the determined displacement distance may be reduced in order to prevent the surgical instrument 119 from reaching a potentially dangerous or uncertain region.

**Fig. 6B** shows a surgical instrument after a single movement of the surgical instrument 119 has been effected according to an embodiment of the invention.

In some embodiments, the target position may have shifted, for example due to movement of fluid or structures in the eye during the single movement of the surgical instrument 119. In such cases, a correction procedure may be performed in order to correct the position of the surgical instrument 119. That is, the sensor 030 may be configured to obtain updated sensor data 030' indicative of the distance between the surgical instrument 119 in the current position (e.g. the position of the surgical instrument 119 after the single movement has been effected) and the surgical target 123. The processor subsystem 040 of the surgical robotic system 100 may then be configured to determine the remaining distance D(remaining) between the surgical instrument 119 and the surgical target 123.

The processor subsystem 040 may then be configured to determine whether the target position is reached, based on the determined remaining distance If the remaining distance between the surgical instrument 119 and the surgical target 123 is determined to be the same, or approximately the same, as the distance between the target position and the surgical target, then the processor subsystem 040 may determine that the target position has indeed been reached. If the remaining distance does not match the distance between the target position and the surgical target, e.g. if the remaining distance does not fall within a predetermined margin, then the processor subsystem 040 may determine that the target position has not been reached. Not reaching the target position may include, for example, overshooting the target position or undershooting the target position.

If the processor subsystem 040 determines that the target position was not reached, the surgical instrument is moved to correct the depth of the surgical instrument. That is, the processor subsystem 040 may determine, based on the determined remaining distance to the surgical target, a correction distance from the target position. The correction distance may be the same as the determined remaining distance, for example if the target position corresponds to the surgical target, but this need not be the case. In some embodiments, the target position is closer to the surgical instrument 119 than the surgical target 123, and the remaining distance between the surgical instrument 119 and the surgical target 123 may not correspond to the correction distance. In such cases, the correction distance may be determined based on the remaining distance. For example, the target position may be a distance *Y* from the surgical target 123, the remaining distance D(remaining) between surgical target 123 and the surgical instrument 119 may be *Y* + Δ*z,* e.g. *D*(*remaining*) = *Y* + *Δz*, so the correction distance may be determined to be *Δz*. That is, the remaining distance may be considered to be the sum of the distance between the target position and the surgical target and the correction distance. If the target position has been overshot, the correction distance may be negative, indicating that the surgical instrument 119 should be retracted by a distance Δ*z*. The correction process may be repeated, if needed, until the target position has been reached.

If, however, the processor subsystem 040 determines that the target position has been reached, it can be determined that the surgical instrument 119 is correctly positioned at the target position.

The correction procedure may be performed in a control mode selected from: a displacement-based control mode, a limited-closed-loop control mode, and a motion-controller-based control mode. In some embodiments, however, the surgical robotic system 100 may be configured with only one or two of these control modes. For example, in some embodiments, the system 100 may be configured to select from only two of these three modes.

### Displacement-based control mode

The displacement-based control mode is analogous to the level of automation and control provided in the initial movement of the surgical instrument 119. That is, a displacement distance is determined (e.g. based on data indicating a target position), a sensory-perceptible representation of the determined displacement distance is output to a user, and the user is requested to confirm the determined displacement distance before the movement of the surgical instrument may occur. That is, in the displacement-based control mode, a displacement distance is determined, this distanced is proposed to and approved by a user (typically a surgeon), and then the movement is automatically executed.

When the displacement-based control mode is selected for the correction procedure, the correction distance (also referred to as a correction displacement distance) is determined as described above. A sensory-perceptible representation of the correction distance is output to the user, and a further confirmation signal is received from the user, indicating a confirmation of the determined correction distance. Upon receiving the further confirmation signal from the user, the surgical instrument 119 is moved over the correction distance in a single (e.g. uninterrupted) movement. That is, the actuator 060 may be controlled to actuate the movable arm part 082 to effect a further single movement of the surgical instrument 119 along the longitudinal axis over the correction displacement distance.

### Limited-closed-loop control mode

In the limited-closed-loop control mode, the control of the actuator 060 is more automated than in the displacement-based control mode and has less user involvement, and may be limited by at least one of the following:
- a predefined time duration for use of the limited-closed-loop control mode,
- a predefined maximum correction displacement distance,
- requiring a certainty score to remain above a certainty score threshold during the correction, and
- requiring a continued user input during the correction, and stopping movement of the surgical instrument upon detecting that the continued user input has ended.

In the limited-closed-loop control mode, the movement of the surgical instrument 119 (e.g. the actuation of the actuator) is determined automatically, for example based on updating sensor data and updating determined distances, e.g. between the surgical instrument 119 and the surgical target 123. In other words, during the movement of the surgical instrument 119 , the sensor 030 may be configured to repetitively obtain updated sensor data 032" indicative of the distance between the surgical instrument 119 and the surgical target 123.

When the limited-closed-loop control mode is time-limited, the automatic movement control of the surgical instrument 119 may be disabled after a preset time. When the limited-closed-loop control mode is displacement-limited, the automatic movement control of the surgical instrument 119 may be disabled once the surgical instrument 119 has been displaced by a preset maximum correction displacement distance.

When the limited-closed-loop control mode depends on a certainty score, the processor 040 may be configured to repeatedly determine a certainty score as described above, e.g. when updated sensor data 032" is obtained, and to compare the updating certainty score to a threshold certainty score. If the certainty score falls below the threshold certainty score, e.g. when the updated sensor data 032" is determined to be too uncertain, the automated control offered during the limited-closed-loop control mode is disabled.

When the limited-closed-loop control mode requires continuous activation, the movement of the surgical instrument 119 may be automated whilst some user input is continuously detected, until the user input is released. For example, the user may press a foot pedal in order to engage the limited-closed-loop control mode, and the movement of the surgical instrument 119 may be automatic until the user releases their foot from the foot pedal. In this example, a continuous user input of a foot pedal is described, although it will be appreciated that this is merely exemplary. In some embodiments, the continuous activation may be provided by gaze detection, gesture capture, voice detection, pressing a trigger or similar button on a controller, or the like. For example, if voice detection is taken as a user input indicating continuous activation, it may be required that the surgeon repeats a phrase at a predetermined frequency, such as once every three or five seconds.

### Motion-controller-based control mode

The motion-controller-based control mode is the least automated control mode of the three control modes described herein, and requires the most user involvement. In the motion-controller-based control mode, the movement of the surgical instrument 119 (e.g. the control of the actuator 060 to actuate the movable arm 082 to effect the movement of the surgical instrument 119) is controlled by the user. The user may provide inputs, such as positioning commands, via e.g. a motion controller or joystick, or similar inputs via the user interface 020. In the motion-controller-based control mode, the user may control the movement of the surgical instrument 119, although effects such as hand tremor and shaking may be reduced or even eliminated using solutions known in the art.

In some embodiments, the control mode to be used when correcting the position of the surgical instrument 119 may be selected by the user, or based on a certainty score calculation. The latter will be described in more detail with reference to Figs. 7A and 7B.

Returning now to Figs. 6A and 6B, in some embodiments, upon determining that the target position has been reached, the process may be repeated. That is, the sensor 030 may obtain updated sensor data 032b indicative of a distance between the surgical instrument 119 and the surgical target. The system 100 may further obtain data indicating a next target position different from the initial target position. For example, the user may indicate a next target position via a graphical user interface. The sensor data 032b obtained with the surgical instrument 119 at this current position may be more reliable than the original sensor data 030, enabling the user to more safely select a target position nearer the surgical target, or indeed select the surgical target as the next target position. An updated displacement distance may then be determined based on the sensor data 032b and the next target position. A sensory-perceptible representation of the updated displacement distance may then be output to the user, e.g. via the user interface. Examples of sensory-perceptible outputs have been provided with reference to Figs. 1 and 5 and earlier, and for the sake of conciseness will not be repeated here. The user may, once again, provide a further confirmation signal indicating a confirmation of the updated displacement distance. Upon receiving the further confirmation signal, the surgical instrument 119 may be moved over the updated displacement distance in a single movement.

This process may be repeated as many times as necessary to reach the surgical target.

Throughout the above description, reference has been made to the movement of the surgical instrument. It is to be understood that the surgical instrument 119 is moved by controlling the actuator 060 to actuate the movable arm part 082, which effects the movement of the surgical instrument 119.

**Fig. 7A** schematically shows a method 700 of selecting a control mode according to an embodiment of the invention. In an operation entitled "CALCULATING CERTAINTY SCORE", the processor subsystem 040 may calculate 710 a certainty score, which may be associated with sensor data obtained by the sensor and/or a distance determined based on the sensor data. For example, a certainty score may be calculated once the surgical instrument 119 has been moved over the determined displacement distance, when determining whether or not the surgical instrument 119 has reached the target position.

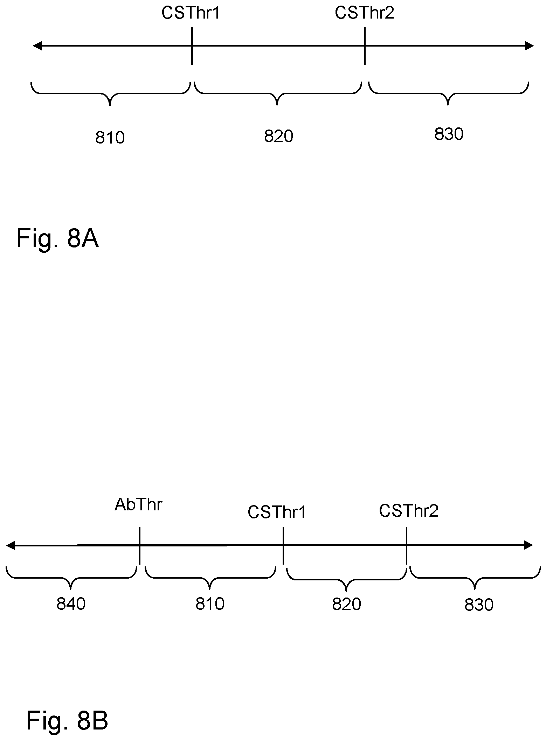

The processor subsystem 040 may then compare 720 the calculated certainty score to a first certainty score threshold, in an operation entitled "COMPARING CERTAINTY SCORE TO FIRST THRESHOLD" of method 700. In some embodiments, the first certainty score threshold may be the only certainty score threshold. This is illustrated in Fig. 7A, described presently. In other embodiments, however, there may be multiple certainty score thresholds, as will be elucidated with reference to Figs. 7B, 8A and 8B.

If, in block 730, it is determined that the calculated certainty score is not higher than the first certainty score threshold, e.g. the measurement or distance associated with the certainty score is not certain or reliable enough, the method 700 proceeds to operation 740, entitled "SELECTING MOTION-CONTROLLER-BASED CONTROL MODE". That is, if the calculated certainty score is not higher than the first certainty score threshold, the actuator may be controlled using the motion-controller-based control mode described above. The user therefore provides the positioning commands, reducing the amount of automation associated with the movement of the surgical instrument 119. This may be due to noisy sensor data, such that the system 100 is unable to detect, with sufficient certainty or confidence, the surgical target.

If, at block 730, the processor subsystem 040 determines that the calculated certainty score exceeds or meets the first certainty score threshold, the method may proceed to either operation 750, entitled "SELECTING DISPLACEMENT-BASED CONTROL MODE", or operation 760, entitled "SELECTING LIMITED-CLOSED-LOOP CONTROL MODE". In some embodiments, the system 100, and specifically the actuator, may be configured to be controlled using one of two control modes when correcting the position of the surgical instrument 119. In some embodiments, the actuator 060 may be controlled either in the motion-controller-based control mode or in the displacement-based control mode. In other words, if the calculated certainty score is below the first certainty score threshold, the actuator 060 may be controlled in the motion-controller-based control mode, and if the calculated certainty score is equal to or above the first certainty score threshold, the actuator 060 may be controlled in the displacement-based control mode. In some embodiments, the actuator 060 may be controlled either in the motion-controller-based control mode or in the limited-closed-loop control mode. That is, if the calculated certainty score is below the first certainty score threshold, the actuator 060 may be controlled in the motion-controller-based control mode, and if the calculated certainty score is equal to or above the first certainty score threshold, the actuator 060 may be controlled in the limited-closed-loop control mode.

Embodiments in which the actuator 060 may be controlled in any of the motion-controller-based control mode, the displacement-based control mode, and the limited-closed-loop control mode will be described with reference to Fig. 7B, 8A and 8B.

**Fig. 7B** schematically shows a variation 700' of the method 700 for selecting a control mode using a certainty score, according to an embodiment of the invention. Fig. 7B differs from Fig. 7A in that a second certainty score threshold, higher than the first certainty score threshold, is used. Operations 710, 720, 730 and 740 and the same as those described with respect to Fig. 7A. For the sake of conciseness, these operations will not be described again here, and only the differences between method 700 of Fig. 7A and the variation 700' of Fig. 7B will be described.

If, in block 730, the processor subsystem 040 determines that the calculated certainty score exceeds the first certainty score threshold, the method 700' proceeds to operation 744, entitled "COMPARING CERTAINTY SCORE TO SECOND THRESHOLD". In operation 744, the calculated certainty score is compared to the second certainty score threshold, which is higher than the first certainty score threshold.

If, in block 746, the processor subsystem 040 determines that the calculated certainty score exceeds the second certainty score threshold, the method 700' may proceed to operation 760, entitled "SELECTING LIMITED-CLOSED-LOOP CONTROL MODE", in which the actuator 060 may be controlled using the limited-closed-loop control mode described above.

If, in block 746, the processor subsystem 040 determines that the calculated certainty score does not exceed the second certainty score threshold, the method 700' may proceed to operation 750, entitled "SELECTING DISPLACEMENT-BASED CONTROL MODE", in which the actuator 060 may be controlled using the displacement-based control mode described above.

Using these certainty score thresholds, the degree of automation of the movement of the surgical instrument 119 may be adapted based on the certainty associated with sensor measurements and/or determined distances. This is illustrated in **Fig. 8A****.**

Referring now to Fig. 8A, when the calculated certainty score falls below the first certainty score threshold CSThr1, the motion-controller-based control mode 810 may be selected. If the calculated certainty score falls above the first certainty score threshold CSThr1 but below the second certainty score threshold CSThr2, the displacement-based control mode 820 is selected. If the calculated certainty score exceeds the second certainty score threshold CSThr2, the limited-closed-loop control mode 830 is selected. As described previously, the use of a second certainty score threshold is optional.

Although not depicted in the figures, in some embodiments, the control mode for the actuator 060 may be selected from the displacement-based control mode and the limited-closed-loop control mode. In other words, the first threshold CSThr1, and similarly operations 720 and 730 may be optional.

Although in the foregoing description, the certainty score has been used to select a control mode, in some embodiments, the comparison of the certainty score to one or more thresholds may also or instead be used to differently control the actuator within a control mode, such as by controlling the maximum displacement or the speed of movement of the surgical instrument 119. For example, the actuator may be operated in the limited-closed-loop control mode, but based on the calculated certainty score, a maximum displacement of the surgical instrument 119 may be used, and/or the speed of the surgical instrument 119 may be determined as a function of the certainty score. The function of the certainty score may be, for example, a stepwise function (e.g., a first speed if the certainty score is below a first threshold, a second speed faster than the first speed if the certainty score is above the first threshold and, optionally, below a second threshold higher than the first threshold, and optionally a third speed faster than the second speed if the certainty score is above the second threshold, and so forth), or a continuous function.

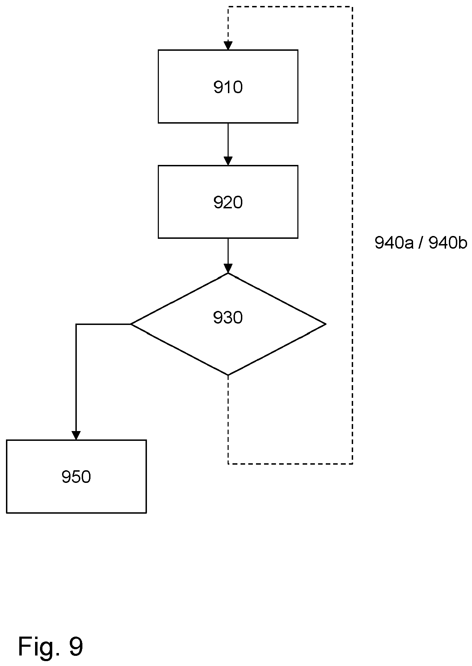

The certainty score may be used in several ways throughout the ocular surgical procedure. To this end, **Fig. 8B** illustrates thresholds for selecting a control mode and/or triggering an abort procedure according to an embodiment of the invention. The abort procedure itself will be described in more detail with reference to Fig. 9.

In addition to, or as an alternative to, the one or more certainty score thresholds described with reference to Figs. 7A, 7B and 8A, a certainty score threshold may be used for triggering an abort procedure. Such a certainty score threshold may be referred to as a critical certainty score threshold, an abort threshold or simply a certainty score threshold. Throughout the following description, the term "abort threshold" will be adopted.

The abort threshold AbThr may be used without any further certainty score thresholds, such as those described with reference to Fig. 8A. That is, in some embodiments, the only certainty score threshold used is the abort threshold AbThr. In some embodiments, however, the abort threshold AbThr may be used in conjunction with at least one further certainty score thresholds CSThr1, CSThr2 for selecting a control mode.

When a certainty score is calculated, such as during a correction procedure or in embodiments in which a certainty score is calculated during the movement of the surgical instrument 119, the calculated certainty score may be compared to an abort threshold AbThr. The abort threshold AbThr may be lower than a threshold CSThr1 used for selecting a control mode. If the calculated certainty score is below the abort threshold AbThr, the abort procedure 840 may be triggered. In some embodiments, the motion-controller-based control mode 810 may be selected if the calculated certainty score falls between the abort threshold AbThr and the first certainty score threshold CSThr1.

**Fig. 9** schematically shows a method 900 for triggering an abort procedure according to an embodiment of the invention.

The method 900 may comprise, in an operation entitled "CALCULATING A CERTAINTY SCORE", calculating 910 a certainty score. This may be done at any point during the ocular surgical procedure, for example when determining whether the surgical instrument 119 has reached the target position, during movement of the surgical instrument 119 or before a movement of the surgical instrument 119. In some embodiments, a certainty score may be calculated whenever sensor data, or updated sensor data, is obtained.

The method may further comprise, in an operation entitled "COMPARING CERTAINTY SCORE TO THRESHOLD", comparing 920 the calculated certainty score to a certainty score threshold, e.g. abort threshold AbThr. If, in block 930, it is determined that the certainty score meets or exceeds the certainty score threshold, the method may continue 940a movement of the surgical instrument, or may continue 940b with the ocular surgical procedure. In some embodiments, such as when sensor data is repeatedly being updated during movement of the surgical instrument 119, the method 900 may be repeated from operation 910, e.g. a new certainty score may be calculated, and so on. The method 900 need not repeat immediately.

If the certainty score falls below the certainty score threshold, the method may proceed to operation 950, entitled "PERFORMING ABORT PROCEDURE".

### Abort procedure

The abort procedure 950 may comprise one or more of the following operations:
- retracting the surgical instrument 119,
- pausing movement of the surgical instrument 119, and
- switching from controlling the actuator in the limited-closed-loop control mode to controlling the actuator in a motion-controller-based control mode.

In particular, pausing movement of the surgical instrument 119 may be performed in combination with either of the other listed operations. In some embodiments, pausing movement of the surgical instrument 119 may be followed by obtaining updated sensor data. In some cases, more reliable sensor data may be obtained whilst the surgical instrument 119 is stationary. In such cases, it may be possible to resume the ocular surgical procedure if, as a consequence of the updated sensor data, an improved certainty score is calculated.

In some embodiments, the abort procedure may comprise first pausing the movement of the surgical instrument 119, obtaining new sensor data, calculating a new certainty score and comparing the new certainty score to the certainty score threshold. If the new certainty score still does not meet the certainty score threshold, the surgical instrument 119 may be retracted.

In some embodiments, the surgical instrument 119 may be retracted immediately upon determining that the abort procedure is triggered.

In some embodiments, the surgical instrument 119 may be partially retracted, e.g. retracted a short distance, such as to return to a previous position of the surgical instrument 119, or fully retracted.

In some embodiments, the control mode for controlling the actuator 060 may be switched if the certainty score is below the abort threshold AbThr. For example, if the actuator is being controlled in the limited-closed-loop control mode and the certainty score falls below the abort threshold AbThr, the control mode of the actuator 060 may be switched to either a displacement-based control mode or a motion-controller-based control mode. If the actuator 060 is being controlled in the displacement-based control mode and the certainty score falls below the abort threshold AbThr, the control mode of the actuator 060 may be switched to a motion-controller-based control mode.

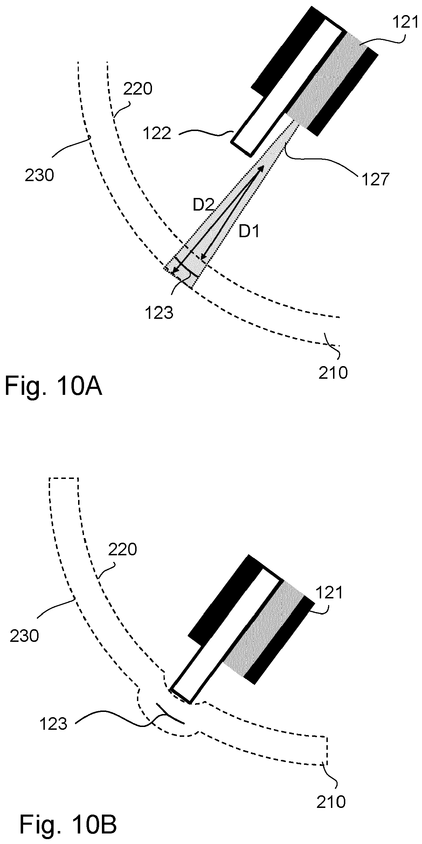

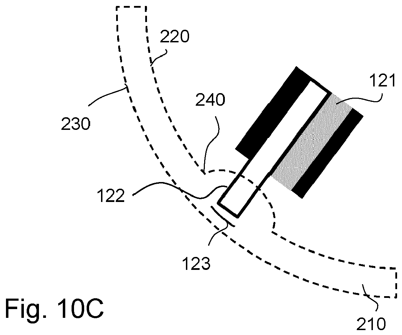

**Fig. 10A** shows a surgical target between two layers of tissue prior to an injection, according to an embodiment of the invention, **Fig. 10B** also shows an indented retina prior to an injection, according to an embodiment of the invention, and Fig. 10C shows a surgical target between two layers of tissue during an injection.

In some embodiments, the sensor 030 may be configured to obtain sensor data 032 indicative of distances between the surgical instrument 119 and multiple layers of tissue. As shown in Fig. 10A, a distance sensor 121, such as an optical fiber, may be attached to, integrated in or coupled to the surgical instrument 119, and may be a component of the sensor 030. That is, the sensor 030 may comprise the distance sensor 121. The sensor 030, e.g. distance sensor 121, may be arranged to determine a first distance, D1, between the surgical instrument 119, e.g. the tip 122 of the surgical instrument 119, and a first layer 220 of tissue in the eye, and a second distance, D2, between the surgical instrument 119, e.g. the tip 122 of the surgical instrument 119, and a second layer 230 of tissue in the eye. Although the arrows indicating distances D1 and D2 are shown in Fig. 10A with different angles, this is merely for ease of illustration, and the distances between the surgical instrument 119 and each layer 220, 230 may be determined along the same axis.

In some embodiments, the surgical target 123 may be known to be between two layers 220, 230 of tissue of the eye, for example by being an intermediate tissue layer. However, when the surgical instrument 119 is moved to contact and, for example, pushes against, the first layer 220 of tissue of the eye, the retina 210 may be indented, causing the surgical target 123 to be displaced or deformed, as illustrated in Fig. 10B. The pressure of surgical instrument on the first layer 220 may result in the indentation of the retina 210, as shown in Fig. 10B. In such cases, the determination of a first distance D1 between the surgical instrument 119 and the first layer 220 of tissue, as well as the determination of a second distance D2 between the surgical instrument 119 and the second layer 230 of tissue, may be used to improve the determination of the position of the surgical target 123. Although not illustrated in Fig. 7B, whilst the surgical instrument 119 is in contact with and/or pressing against a layer 220 of tissue, the sensor 030, e.g. distance sensor 121, may be configured to obtain sensor data indicating a distance D1 between the surgical instrument 119 and the first layer 220 of tissue, and indicating a distance D2 between the surgical instrument 119 and the second layer 230 of tissue.

In some embodiments, the system 100 may detect when the surgical instrument 119 reaches the first layer 220. For example, the target position may be determined to correspond to the first layer 220. Once this position has been reached, the actuator 060 may be controlled to retract the surgical instrument 119 a distance x, before advancing the surgical instrument 119, e.g. a distance 2x, preferably at a higher speed, in order to improve the piercing/displacement ratio, and thereby reduce the indentation of the retina. The resulting displacement of the surgical instrument 119 would therefore be a distance of x from the starting position. That is, the penetration of a layer of tissue may be done by advancing the surgical instrument 119 at a higher speed, e.g. in a fast piercing motion. The speed used for the fast piercing motion may be preset, or the maximum speed of the surgical instrument 119 may be used.

In some embodiments, a bleb 240 may be formed upon injecting a fluid into the retina 210, as illustrated in Fig. 10C. During an injection, the position of the surgical instrument 119 may be corrected. That is, whilst the surgical instrument 119 is injecting fluid between these two layers 220 and 230, the center of the bleb 240 may be determined, e.g. based on determining the distances between the surgical instrument 119 and each of the two layers 220 and 230, respectively. The surgical instrument 119 may be retracted or extended to the bleb center.

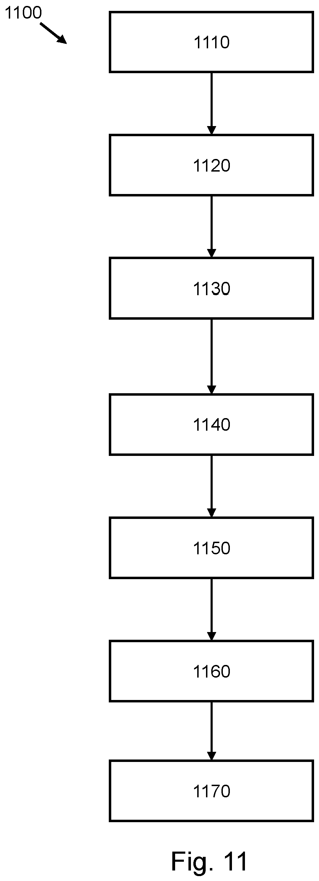

**Fig. 11** schematically illustrates a method 1100 for controlling a surgical robotic system during use in an ocular surgical procedure, the surgical robotic system comprising a surgical arm, the surgical arm comprising a movable arm part, the movable arm part comprising an instrument connector for mounting of a surgical instrument having a longitudinal axis, the movable arm part having at least one degree of freedom to enable longitudinal movement of the surgical instrument along the longitudinal axis of the surgical instrument towards or away from an ocular surgical target, the surgical robotic system further comprising an actuator configured and arranged for actuating the movable arm part to effect the longitudinal movement of the surgical instrument. The method 1100 comprises, in an operation titled "OBTAINING SENSOR DATA", obtaining 1110 sensor data indicating a distance between the surgical instrument and the surgical target, when the surgical instrument is at an initial position. The method 1100 further comprises, in an operation entitled "DETERMINING INITIAL DISTANCE", determining 1120 an initial distance between the surgical instrument at the initial position and the surgical target based on the sensor data. The method 1100 further comprises, in an operation entitled "OBTAINING TARGET POSITION RELATIVE TO SURGICAL TARGET", obtaining 1130 data indicative of a target position of the surgical instrument relative to the surgical target. The method 1100 further comprises, in an operation entitled "DETERMINING DISPLACEMENT DISTANCE", determining 1140 a displacement distance for the surgical instrument based on the sensor data and the target position. The method 1100 further comprises, in an operation entitled "OUTPUTTING DISPLACEMENT DISTANCE", outputting 1150, to the user, a sensory-perceptible representation of the displacement distance. The method 1100 further comprises, in an operation entitled "RECEIVING USER CONFIRMATION", receiving 1160, from the user, a confirmation signal. The method 1100 further comprises, in an operation entitled "ACTUATING MOVABLE ARM", upon receiving the confirmation signal, controlling 1170 the actuator to actuate the movable arm part to effect a single movement of the surgical instrument along the longitudinal axis over the displacement distance. It is noted that the operations of the method 1100 need not be performed strictly sequentially. For example, operations 1120 and 1130 may be performed in a different order or substantially simultaneously. Similarly, operations 1110 and 1120 may be performed in a different order or substantially simultaneously.

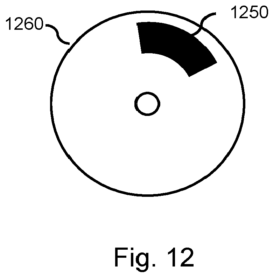

The method according to the invention may be implemented on a processor as a computer implemented method, or in dedicated hardware, or in a combination of both. Executable code for a method according to the invention may be stored on a computer program product. Examples of computer program products include memory devices, optical storage devices, integrated circuits, servers, online software, etc. **Fig. 12** shows a computer program product in the form of an computer readable medium 1260 which comprises non-transitory program code 1250 for causing a processor to perform a method according to the invention when said program code is executed the processor.

In a preferred embodiment, the computer program comprises computer program code means adapted to perform all the steps of a method according to the invention when the computer program is executed by a processor. Preferably, the computer program is embodied on a computer readable medium.

Throughout the above description, reference is made to various distances between the surgical instrument and a target position, surgical target, layer of tissue, or the like. These may correspond to the distance between the tip of the surgical instrument and the target position, surgical target, layer of tissue, etc., respectively. That is, distances mentioned throughout this description may be taken from the tip of the surgical instruments, according to some embodiments.

It should be noted that the above-mentioned embodiments illustrate rather than limit the invention, and that those skilled in the art will be able to design many alternative embodiments.

In the claims or clauses, any reference signs placed between parentheses shall not be construed as limiting the claim or clause. Use of the verb "comprise" and its conjugations does not exclude the presence of elements or steps other than those stated in a claim or clause. The article "a" or "an" preceding an element does not exclude the presence of a plurality of such elements. The invention may be implemented by means of hardware comprising several distinct elements, and by means of a suitably programmed computer. In the device claim or clause enumerating several means, several of these means may be embodied by one and the same item of hardware. The mere fact that certain measures are recited in mutually different dependent claims or clauses does not indicate that a combination of these measures cannot be used to advantage.

## Claims

1. A surgical robotic system (100) for use in an ocular surgical procedure, comprising:
- a surgical arm (080) comprising a movable arm part (082), the movable arm part (082) comprising an instrument connector for mounting of a surgical instrument (119) having a longitudinal axis, the movable arm part (082) having at least one degree of freedom to enable longitudinal movement of the surgical instrument (119) along the longitudinal axis of the surgical instrument (119) towards or away from an ocular surgical target (123);
- a sensor (030) configured to obtain sensor data (032) indicating a distance between the surgical instrument (119) and the surgical target (123);
- a user interface (020) configured to receive user input from a user and to output sensory perceptible output to the user;
- an actuator (060) configured and arranged for actuating the movable arm part (082) to effect the longitudinal movement of the surgical instrument (119);
- a processor subsystem (040) configured to:
- at an initial position of the surgical instrument (119), obtain sensor data (032) from the sensor (030) and determine an initial distance between the surgical instrument (119) and the surgical target (123) based on the sensor data (032);
- obtain data indicative of a target position of the surgical instrument (119) relative to the surgical target (123);
- determine a displacement distance for the surgical instrument (119) based on the sensor data (032) and the target position;
- output, via the user interface (020), a sensory-perceptible representation of the determined displacement distance;
- receive, via the user interface (020), a confirmation signal from the user; and
- upon receiving the confirmation signal, control the actuator (060) to actuate the movable arm part (082) to effect a single movement of the surgical instrument (119) along the longitudinal axis over the determined displacement distance.

2. The surgical robotic system (100) of claim 1, wherein the processor subsystem (040) is further configured to:
- upon completing the single movement of the surgical instrument (119), obtain updated sensor data (032') from the sensor (030) and determine a remaining distance between the surgical instrument (119) and the surgical target (123) based on the updated sensor data (032');
- determine whether the target position is reached based on the determined remaining distance; and
- if not, control the actuator (060) to correct a depth of the surgical instrument (119) based on the determined remaining distance.

3. The surgical robotic system (100) of claim 2, wherein the processor subsystem (040) is configured to control the actuator (060) in a displacement-based control mode to correct the depth of the surgical instrument (119) by:
- determining a correction displacement distance to reach the target position from the determined remaining distance to the surgical target (123);
- outputting, via the user interface (020), a sensory-perceptible representation of the correction displacement distance;
- receiving, via the user interface (020), a further confirmation signal from the user; and
- upon receiving the further confirmation signal, controlling the actuator (060) to actuate the movable arm part (082) to effect a further single movement of the surgical instrument (119) along the longitudinal axis over the correction displacement distance.

4. The surgical robotic system (100) of claim 2 or 3, wherein the processor subsystem (040) is configured to control the actuator (060) in a limited-closed-loop control mode in which updated sensor data (032) is used to correct the depth of the surgical instrument (119), wherein the limited-closed-loop control mode is limited by at least one of:
- a predefined time duration for use of the limited-closed-loop control mode;
- a predefined maximum correction displacement distance;
- requiring a certainty score to remain above a certainty score threshold during the correction, the certainty score representing a certainty of the determined remaining distance; and
- requiring a continued user input during the correction, and stopping movement of the surgical instrument (119) upon detecting that the continued user input has ended.

5. The surgical robotic system (100) of any preceding claim, wherein the processor subsystem (040) is further configured to:
- calculate a certainty score representing a certainty of a determined distance between the surgical instrument (119) and the surgical target (123); and
- adapt the control of the actuator (060) according to the calculated certainty score.

6. The surgical robotic system (100) of claim 5, wherein the processor subsystem (040) is further configured to calculate the certainty score based on at least one of: an estimate of measurement noise of the sensor (032), a detection of a presence of a dynamic movement of the surgical target (123), a comparison of the sensor data (032) with a model representing a reference for the sensor data (032), and a comparison between an estimated surgical target position and a predicted surgical target position, wherein the predicted surgical target position is based on instrument movements as measured by at least one position sensor.

7. The surgical robotic system (100) of claim 5, dependent on claim 4, wherein the processor subsystem (040) is configured to calculate the certainty score repeatedly, and to perform an abort procedure if the calculated certainty score falls below a certainty score threshold,
wherein the abort procedure comprises at least one of:
- retracting the surgical instrument (119);
- pausing movement the surgical instrument (119); and
- switching from controlling the actuator (060) in the limited-closed-loop control mode to controlling the actuator (060) in a motion-controller-based control mode, in which the processor subsystem (040) is configured to control the actuator (060) according to position control commands received from the user via a motion controller.

8. The surgical robotic system (100) of claim 5 or 6, wherein the processor subsystem (040) is further configured to select a control mode from a set of control modes based on the calculated certainty score, the set of control modes comprising at least two of: a motion-controller-based control mode, a displacement-based control mode and a limited-closed-loop control mode, wherein:
- in the motion-controller-based control mode, the processor subsystem (040) is configured to control the actuator (060) according to position control commands received from the user via a motion controller of the user interface (020);
- in the displacement-based control mode, the processor subsystem (040) is configured to:
- determine a correction displacement distance to reach the target position from the determined remaining distance to the surgical target (123);
- output, via the user interface (020), a sensory-perceptible representation of the correction displacement distance;
- receive, via the user interface (020), a further confirmation signal from the user; and
- upon receiving the further confirmation signal, control the actuator (060) to actuate the movable arm part (082) to effect a further single movement of the surgical instrument (119) along the longitudinal axis over the correction displacement distance; and
- in the limited-closed-loop control mode, the processor subsystem (040) is configured to be limited by at least one of:
- a predefined time duration for use of the limited-closed-loop control mode;
- a predefined maximum correction displacement distance;
- requiring a certainty score to remain above a certainty score threshold during the correction, the certainty score representing a certainty of the determined remaining distance; and
- requiring a continued user input during the correction, and stopping movement of the surgical instrument (119) upon detecting that the continued user input has ended.

9. The surgical robotic system (100) of any one of claims 5 to 8, wherein the processor subsystem (040) is further configured to output, via the user interface (020), a sensory-perceptible representation of the calculated certainty score, prior to receiving the confirmation signal from the user.

10. The surgical robotic system (100) of any preceding claim, wherein the sensor (030) comprises at least one of:
- an optical coherence tomography (OCT) sensor configured to optically couple to an optical fiber attached to or integrated in the surgical instrument (119);
- intraoperative optical coherence tomography (iOCT) through a microscope;
- a stereo camera through a microscope;
- an optical interferometric sensor integrated in or attached to the surgical instrument (119);
- a time-of-flight sensor integrated in or attached to the surgical instrument (119); and
- an ultrasonic sensor integrated in or attached to the surgical instrument (119).

11. The surgical robotic system (100) of any preceding claim, wherein the processor subsystem (040) is further configured to:
- obtain sensor data (032) indicative of a distance between a first layer (220) of tissue of an eye and a second layer (230) of tissue of the eye, wherein the surgical target (123) is between the first layer (220) and the second layer (230); and
- correct the position of the surgical instrument (119) such that the surgical instrument (119) is between the first layer (220) and the second layer (230).

12. The surgical robotic system (100) of any preceding claim, wherein the processor subsystem (040) is further configured to obtain sensor data (032) indicative of a distance between a first layer (220) of tissue of an eye and a second layer (230) of tissue of the eye, wherein the surgical target (123) is between the first layer (220) and the second layer (230); and
wherein the data indicative of a target position of the surgical instrument (119) relative to the surgical target (123) comprises data indicative of the distance between the first layer (220) and the second layer (230).

13. A computer-implemented method (1100) for controlling a surgical robotic system (100) during use in an ocular surgical procedure, the surgical robotic system (100) comprising a surgical arm (080), the surgical arm (080) comprising a movable arm part (082), the movable arm part (082) comprising an instrument connector for mounting of a surgical instrument (119) having a longitudinal axis, the movable arm part (082) having at least one degree of freedom to enable longitudinal movement of the surgical instrument (119) along the longitudinal axis of the surgical instrument (119) towards or away from an ocular surgical target (123), the surgical robotic system (100) further comprising an actuator (060) configured and arranged for actuating the movable arm part (082) to effect the longitudinal movement of the surgical instrument (119), the method comprising:
- at an initial position of the surgical instrument, obtaining (1110) sensor data (032) indicating a distance between the surgical instrument (119) and the surgical target (123);
- determining (1120) an initial distance between the surgical instrument (119) at the initial position and the surgical target (123) based on the sensor data (032);
- obtaining (1130) data indicative of a target position of the surgical instrument (119) relative to the surgical target (123);
- determining (1140) a displacement distance for the surgical instrument (119) based on the sensor data (032) and the target position;
- outputting (1150), to the user, a sensory-perceptible representation of the displacement distance;
- receiving (1160), from the user, a confirmation signal; and
- upon receiving the confirmation signal, controlling (1170) the actuator (060) to actuate the movable arm part (082) to effect a single movement of the surgical instrument (119) along the longitudinal axis over the displacement distance.

14. A computer-readable storage medium (1260) comprising transitory or non-transitory data representing a computer program (1250), the computer program comprising instructions for causing a processor system to carry out the method of claim 13.
